# EUROPÄISCHE PATENTSCHRIFT

(11) **EP 0 623 651 B1**
(45) Veröffentlichungstag und Bekanntmachung des Hinweises auf die Patenterteilung: **29.12.1997**
(21) Anmeldenummer: 94106978.3
(22) Anmeldetag: 03.05.1994
(51) Int. Cl.: C08L 53/02, C08L 23/10, C08L 91/00, A61M 1/00

(54) **Thermoplastisches Elastomercompound**
Thermoplastic elastomer compound
Composé d'élastomère thermoplastique

(30) Priorität: 06.05.1993 DE 4315003
(43) Veröffentlichungstag der Anmeldung: 09.11.1994
(73) Patentinhaber: THEKA-flex Produktions- und Handels- GmbH, D-21357 Bardowick (DE)
(72) Erfinder: Schäfer, Helmut, D-21218 Seevetal (DE)
(74) Vertreter: Schaefer, Konrad, Dipl.-Phys.

(56) Entgegenhaltungen:
- EP-A- 0 452 089
- DE-A- 3 337 997
- US-A- 3 865 776
- PATENT ABSTRACTS OF JAPAN vol. 12, no. 279 (C-517) 1. August 1988 & JP-A-63 057 661 (ASAHI CHEM. IND. CO.) 12. März 1988

## Beschreibung

Die Erfindung bezieht sich auf ein thermoplastisches Elastomercompound nach dem Oberbegriff des Anspruches 1 sowie auf aus diesem Compound hergestellte, insbesondere für medizinische Zwecke geeignete Kunststoffartikel.

Viele medizinische Artikel, z.B. Infusionsschläuche aber auch eine ganze Reihe weiterer Artikel, bestehen aus Kunststoffen, die gleichzeitig transparent und elastisch sind. Gängigerweise handelt es sich dabei um weichgemachtes Polyvinylchlorid (Weich-PVC), dem durch Zusatz von z.B. Dicarbonsäureestern als Weichmachern die notwendige Elastizität verliehen wird. Die Verwendung von PVC ist jedoch insbesondere um Hinblick auf die eingesetzten Weichmacher physiologisch nicht unbedenklich. So kann es dazu kommen, daß die Dicarbonsäureester aus dem PVC in ein kontaktierendes Medium emigrieren und z.B. im Falle von Infusionsschläuchen in den Körper des Patienten gelangen, wo sie gegebenenfalls zu toxischen Substanzen metabolisiert werden. Es wird darum in letzter Zeit verstärkt nach einem Ersatz für weichgemachtes PVC im medizinischen Bereich gesucht.

Rein theoretisch würden sich hierfür die seit längerer Zeit bekannten Blockcopolymere, z.B. Styrolethylenbutylenstyrol (SEBS) eignen. Ein typisches SEBS-Makromolekül enthält zu 20 bis 50% Styrolblöcke. Die Styrolblöcke haben ein Molekulargewicht, das sich in einem Bereich zwischen 5.000 bis 40.000 bewegt. Der Ethylen-Butylen-Block besitzt ein Molekulargewicht, das zwischen 20.000 und 500.000 variieren kann.

SEBS- Blockcopolymere (und auch das seit kürzerer Zeit bekannte (SPBS) Styrolpropylenbutylenstyrol) besitzen elastomere Eigenschaften und lassen sich theoretisch zu transparenten Endprodukten verarbeiten. Allerdings wird z.B. SEBS in der Regel nicht in reiner Form verarbeitet. Dem steht zum einen entgegen, daß sich reines SEBS z.B. beim Einsatz im Spritzgußverfahren nur relativ schwierig ausformen läßt. Zum anderen sind in der Regel zusätzliche Eigenschaften, wie Reißfestigkeit, Hitzestabilität etc. erwünscht, die durch reines SEBS nicht dargestellt werden können. Daher werden Blockcopolymeren, wie z.B. SEBS, in der Regel weitere Substanzen zugegeben, die im Hinblick auf die zu erreichenden Eigenschaften erwünscht sind.

Diesbezüglich offenbait z.B. die US-PS 4,386,179 ein im Extruder hergestelltes Gemisch (Compound) aus Blockcopolymer und Polysiloxanen. Aus einem derartigen Compound lassen sich z.B. Folien mit extremer Reißfestigkeit herstellen. In der gleichen Schrift wird weiterhin auf die US-PS 4,041,103 verwiesen, die ein Compound aus SEBS mit Polyamid offenbart. Aus diesem Compound sollen sich besonders hitzebeständige Kunststoffe herstellen lassen.

Weiterhin sind z.B. auch Compounds, bestehend aus Polypropylen und SEBS, z.B. von Gupta et al (Journal of Applied Polymer Science, Vol. 29, 3513-3531 (1984)) beschrieben worden. Hier ging es allerdings eher darum die Schlagfestigkeit des Polypropylens zu erhöhen.

Es ist fraglich, ob die bislang bekannten, auf Basis von Blockcopolymeren erhaltenen Compounds die z.B. für medizinische Zwecke gewünschten Eingenschaften besitzen. So läßt sich z.B. das zuletzt genannte Compound aus SEBS und Polypropylen zwar gut verarbeiten, ermöglicht jedoch nicht die Herstellung vom Produkten mit hoher Transparenz.

Weitere Compounds sind aus der DE-A-33 37 997 und der EP-A-0452089 bekannt. Die DE 33 37 997 betrifft eine Kunstharzzusammensetzung aus einem hydrierten Blockcopolymer, wie z.B. SEBS, einem Äthylen/Acrylatcopolymer und einem isotaktischen Propylen. Derartige Kunstharze eignen sich insbesondere zur Herstellung wenig flexibler Folien mit hoher Festigkeit, wie sie z.B. bei Blutbeuteln Verwendung finden. Die EP 0452089 betrifft dagegen ein thermoplastisches Elastomer, das als Bestandteile ein hydriertes Blockcopolymer, einen olefinischen teilweise vernetzten Copolymerkautschuk, Polyolefin und einen Weichmacher vom Mineralöltyp aufweist.

Ausgehend davon, ist es Aufgabe der Erfindung, ein thermoplastisches Elastomercompound zu schaffen, das sich besonders einfach herstellen läßt und das sich zu transparenten Kunststoffprodukten mit gut einstellbarer Elastizität in einem Bereich weiterverarbeiten läßt, der von hochflexibel bis halbhart reicht.

Gelöst wird diese Aufgabe durch ein Thermoplastisches Elastomercompound, enthaltend ein Blockcopolymer, einen Zusatzstoff auf Polypropylenbasis und einen Weichmacher, **dadurch gekennzeichnet,** daß es ausschließlich folgende Komponenten enthält:
- 1.: als Blockcopolymer 1 Teil SEBS oder 1 Teil SPBS
- 2.: als Zusatzstoff 0,1 bis 1 Teil Polypropylenrandomcopolymer, das als Comonomer Ethylen oder Butylen in Konzentrationen von 2 % bis 8 % enthält.
- 3.: als Weichmacher 0,1 bis 1 Teil eines Öles, das höchstens 10 % aromatische Anteile enthält und
- 4.: gegebenenfalls ein Antistatikum mit einem Gewichtsanteil von 0,1 % bis 0,5 %,
wobei die Herstellung des Elastomercompounds unter Ausschluß einer chemischen Vernetzung erfolgt.

Der Begriff Randomcopolymer ist in der Kunststofftechnik gängig und bedeutet gemäß Saechtling Kunststoff-Taschenbuch, 23. Ausgabe, Seite 48, daß die einzelnen Monomere "statistisch regellos in den Makromolekülen verteilt sind". Derartige Randomcopolymere lassen sich durch eine entsprechende Steuerung des Polymerisationsvorganges ohne weiteres herstellen und sind überdies auf dein Markt erhältlich (z.B von der Firma "Neste Chemicals" unter dem Namen "Trandpalene")

Es hat sich herausgestellt, daß zwar generell Zusatzstoffe auf Polypropylenbasis die Verarbeitbarkeit eines Compounds, das SEBS oder SPBS enthält, erleichtern.

Jedoch führt ausschließlich die Verwendung eines Polypropylenrandomcopolymers zu einem Compound, aus dem sich Kunststoffprodukte mit der insbesondere auf dem medizinischen Sektor erforderlichen Transparenz herstellen lassen.

Ein weiteres Merkmal ist, daß bei Herstellung des eifindungsgemäßen Elastomercompounds ausdrücklich auf den Einsatz chemischer Vernetzungsmittel verzichtet wird, d.h. eine chemische Vernetzung der eingesetzten Komponenten nicht erfolgt und zur Herstellung eines Compounds mit den gewünschten Eigenschaften, wie sich herausgestellt hat, nicht erforderlich ist.

Erfindungsgemäß ist vorgesehen, daß sich das Compound aus einem Teil SEBS bzw. SPBS, 0,1 bis 1 Teil Polypropylenrandomcopolymer und 0,1 bis 1 Teil des als Weichmacher verwendeten Öles zusammensetzt. Das dermaßen zusammengesetzte erfinderische Compound läßt sich problemlos weiterverarbeiten. Durch Auswahl definierter Zusammensetzungen innerhalb der vorgegebenen Bereichsgrenzen läßt sich insbesondere die Elastizität und die Shorehärte der aus dem Compound hergestellten Kunststoffprodukte genau einstellen.

Die als Weichmacher verwendeten Öle liegen in der Regel gut in dem Kunststoffcompound gebunden vor. Dies gewährleistet, daß die Elastizität der Kunststoffprodukte auch über einen längeren Zeitraum erhalten bleibt.

Vorteilhafte Ausgestaltungen der Erfindung sind in den Unteransprüchen geschützt.

So betrifft Anspruch 2 eine Ausgestaltung des erfindungsgemäßen Compounds, bei dem als Weichmacher ein Öl frei von Aromatenanteilen verwendet wird (und sich aus 25% bis 45% naphtenischen Anteilen und 75% bis 55% paraffinischen Anteilen zusammensetzt). Bei Vorliegen von Aromaten besteht die Gefahr, daß Kunststoffe mit Farbstichigkeit erhalten werden, die für bestimmte Einsatzzwecke nicht geeignet sind. Dies läßt sich durch Verwendung aromatenfreier Weichmacheröle in vorteilhafter Weise ausschließen.

Insbesondere im Hinblick auf medizinische Kunststoffprodukte ist in diesem Zusammenhang gemäß Anspruch 3 vorgesehen, daß es sich bei dem als Weichmacher verwendeten Öl um Paraffinum perliquidum nach DAB 10 handelt. Bei Verwendung eines derartigen Weichmacheröles erhält man Kunststoffcompounds, aus denen sich wasserklare Kunststoffartikel herstellen lassen, die höchste medizinische Standards erfüllen.

Es kann weiterhin vorgesehen sein, daß dem erfindungsgemäßen Compound ein Antistatikum in Anteilen von 0,1% bis 0,5% zugesetzt wird. Es kann sich um ethoxilierte Fettamine oder Fettsäureester aus gesättigten Fettsäuren (C10 bis 24) mit physiologisch unbedenklichen mehrwertigen Alkoholen handeln. Aus mit Antistatikum versehenen Compounds lassen sich Kunststoffartikel herstellen, die sich nicht elektrostatisch aufladen und daher zu geringerer Verschmutzung neigen. Auch dies ist insbesondere auf dem medizinischen Sektor von Vorteil.

Theoretisch ist das erfindungsgemäße Compound zur Herstellung von Artikeln auf unterschiedlichen Bereichen, also auch im technischen Bereich, geeignet. Vorteilhafterweise wird es jedoch insbesondere auf dem medizinischen Sektor, insbesondere als Ersatz für das bislang verwendete weichgemachte PVC eingesetzt.

Gemäß Anspruch 4 sind vorteilhafterweise medizinische Artikel für folgende Gebiete aus dem erfindungsgemäßen Compound hergestellt: Infusion, Transfusion, Dialyse, enterale Ernährung, Laparoskopie und Blutentnahme. Die Herstellung der jeweiligen Artikel, die in der Regel Einmalartikel sind, erfolgt durch Extrusion oder Spritzguß.

Vorteilhafteiweise handelt es sich bei derartigen medizinischen Artikeln um Schläuche, mit denen Flüssigkeiten in den menschlichen Körper eingeleitet bzw. daraus abgeleitet werden. Dies können z.B. Infusionsschläuche, aber auch Schlauchsysteme sein, die z.B. bei der Dialyse verwendet werden. Es kann sich auch um Schlauchsysteme handeln, mit denen im Zuge der Laparoskopie das Operationsfeld gespült wird etc.

Die Erfindung ist selbstverständlich nicht auf die speziell genannten medizinischen Artikel beschränkt. Aus dein erfindungsgemäßen Compound können vielmehr sämtliche Artikel, insbesondere Einmalartikel, die bislang aus weichgemachten PVC bestehen, hergestellt werden. Die gewünschten Shorehärten bzw. das gewünschte Elastizitätsverhalten, Reßfestigkeit etc. lassen sich durch Variation der einzelnen, in dem Compound enthaltenen Substanzen exakt einstellen.

Ein Sonderfall enies medizinischen Artikels, der sich aus dem erfindungsgemäßen Compound herstellern läßt, soll hier besondere Erwälmung finden. Es handelt sich dabei um eine sogenannte Blutbeobachtungskammer, wie sie in jüngst bekannt gewordenen Vakuumblutentnahmevorrrichtungen eingesetzt werden kann (siehe z.B. PCT/EP 90/02243). Derartige Vakuumblutentnahmevorrichtungen arbeiten grundsätzlich mit einem evakuierten Röhrchen, das mit einem Stopfen verschlossen ist, und mit einer beidseitig zugespitzten Kanüle. Zur Blutentnahme wird das distale Ende der Kanüle in die Vene des Patienten gestochen und das proximale Ende durch den Stopfen in das evakuierte Röhrchen eingesetzt. Aufgrund des Vakuums wird dann die Blutprobe angesogen. Um Hämatome infolge von Fehlpunktionen zu vermeiden, ist nun in den jüngst bekannt gewordenen Systemen eine transparente Blutbeobachtungskammer in Stichrichtung vor dem Stopfen an dem Röhrchen angeordnet. Bei der Blutentnahme wird das proximale Ende der Kanüle zunächst nur bis in diese Kammer eingeführt und dann die Vene punktiert. Wurde die Vene getroffen, dann tritt aufgrund des Veneninnendruckes in der Blutbeobachtungskammer Blut aus, was sich optisch überprüfen läßt. Die Blutentnahme kann dann durch weiteren Vorschub der proximalen Kanülenspitze in das evakuierte Röhrchen fortgesetzt werden.

Es ist einsichtig, daß die Blutbeobachtungskammer einen besonders hohen Grad an Transparenz aufweisen muß, der durch das erfindungsgemäße Compound gewährleistet wird. Darüber hinaus hat sich herausgestellt, daß bei Herstellung der Blutbeobachtungskammer aus einem mit Antistatikum ausgerüsteten Compound das Blut flächig an der Innenwand der Kammer absorbiert wird. Selbst bei geringen Blutmengen hat man daher einen eindeutigen optischen Effekt in der Kammer.

Im folgenden soll die Erfindung anhand mehrerer Beispiele erläutert werden.

### Beispiel 1: Herstellung eines Compounds.

Es werden 100 Teile SEBS bzw. SPBS mit 80 Teilen Paraffinum perliquidum und 60 Teilen Polypropylenrandomcopolymer in einem Doppelschneckenextruder mit gleichsinnig 280 Upm drehenden Schnecken (L/D Verhältnis von 25:1) bei 200°C und 140 bar kontinuierlich geknetet und gemischt. Der Durchsatz beträgt 250 kg pro Stunde Das aus dein Extruder austretende Gemisch wird heiß granuliert und läßt sich zu Kunststoffartikeln mit einer Härte Shore A von ca. 80° verarbeiten.

### Beispiel 2: Herstellung eines Infusionsschlauches aus dem Compound gemäß Beispiel 1.

Das Compound wird in einen herkömmlichen Einschneckenextruder (L/D-Verhältnis 27:1) eingefüllt und dort mit 75 Upm bei 80 bar und 180° C extrudiert. Die Extruderleistung ist dabei so eingestellt, daß pro Minute 180 m herkömmlicher Infusionsschlauch gebildet werden.

### Beispiel 3: Herstellung eines weiteren Compounds.

Die Herstellung erfolgt, wie in Beispiel 1. Es werden jedoch zusätzlich 0,2 Anteile Antistatikum (z.B. ein von der Firma Henkel unter dem Namen Loxiol G-12 hergestelltes Monodiglyceridgemisch) zugesetzt. Ansonsten erfolgt die Herstellung wie im Beispiel 1. Das so hergestellte Compound wird dann wieder heiß granuliert und kann zur Weiterverarbeitung in einer herkömmlichen Spritzgießmaschine plastifiziert und in einem geeigneten Spritzwerkzeug geformt werden. Auf diese Weise läßt sich z.B. die oben beschriebene Blutbeobachtungskammer herstellen.

Die Erfindung ist selbstverständlich nicht auf die genannten Beispiele beschränkt. Wie oben ausgeführt, lassen sich durch entsprechende Auswahl der Zusammensetzung des Compounds bzw. der Bedingungen für die Weiterverarbeitung des Compounds eine ganze Reihe von auf dem medizinischen Gebiet einsetzbaren Altikeln herstellen. Es ist weiterhin auch denkbar, das erfindungsgemäße Compound zur Herstellung von Membranen einzusetzen, deren z.B. Elastizität besonders gut einstellbar ist.

In der Regel kann davon ausgegangen werden, daß das erfindungsgemäße Compound anstelle des bislang verwendeten weichgemachten PVC unter anderem vorteilhaft im medizinischen Bereich eingesetzt werden kann.

## Patentansprüche

1. Thermoplastisches Elastomercompound, enthaltend ein Blockcopolymer, einen Zusatzstoff auf Polypropylenbasis und einen Weichmacher, **dadurch gekennzeichnet,** daß es ausschließlich folgende Komponenten enthält:
1. als Blockcopolymer 1 Teil SEBS oder 1 Teil SPBS
2. als Zusatzstoff 0,1 bis 1 Teil Polypropylenrandomcopolymer, das als Comonomer Ethylen oder Butylen in Konzentrationen von 2 % bis 8 % enthält.
3. als Weichmacher 0,1 bis 1 Teil eines Oles, das höchstens 10 % aromatische Anteile enthält und
4. gegebenenfalls ein Antistatikum mit einem Gewichtsanteil von 0,1 % bis 0,5 %,
wobei die Herstellung des Elastomercompounds unter Ausschluß einer chemischen Vernetzung erfolgt.

2. Compound nach Anspruch 1, **dadurch gekennzeichnet,** daß der in ihm enthaltene Weichmacher ein Öl mit naphtenischen Anteilen in einem Bereich von 25% bis 45% und paraffinischen Anteilen in einem Bereich von 55% bis 75% ist und frei von Aromatenanteilen ist.

3. Compound nach Anspruch 2, **dadurch gekennzeichnet,** daß als Weichmacher Paraffinum perliquidum nach DAB 10 enthalten ist.

4. Medizinische Artikel für die Gebiete Infusion, Transfusion, Dialyse, enterale Ernährung, Laparoskopie und Blutentnahme,dadurch gekennzeichnet, daß sie durch Extrusion oder Spritzguß aus einem Compound gemäß den Ansprüchen 2 bis 3 herstellbar sind.

5. Medizinische Artikel nach Anspruch 4, **dadurch gekennzeichnet,** daß es sich um Schläuche bzw. Schlauchsysteme handelt.

## Claims

1. A thermoplastic elastomer compound, containing a block copolymer, a polypropylene-based additive, and a plasticiser, characterised in that it contains only the following components:
1. As block copolymer: 1 part SEBS or 1 part SPBS
2. As additive: 0.1 to 1 part polypropylene random copolymer which contains, as comonomer, ethylene or butylene in concentrations of 2% to 8%.
3. As plasticiser: 0.1 to 1 part of an oil containing not more than 10% aromatic constituents and
4. If required, an antistatic having a content by weight of 0.1% to 0.5%,
the production of the elastomer compound being effected to the exclusion of any chemical crosslinking.

2. A compound according to claim 1, characterised in that the plasticiser contained therein is an oil having naphthenic constituents in a range from 25% to 45% and paraffinic constituents in a range from 55% to 75% and is free of aromatic constituents.

3. A compound according to claim 2, characterised in that it contains as plasticiser paraffinum perliquidum to DAB 10.

4. Medical articles for the following areas: infusion, transfusion, dialysis, enteral nutrition, laparoscopy and blood sampling, characterised in that they can be produced by extrusion or injection moulding from a compound according to claims 2 to 3.

5. Medical articles according to claim 4, characterised in that flexible tubes or flexible tube systems are involved.

## Revendications

1. Composé d'élastomère thermoplastique contenant un copolymère en masse, un adjuvant à base de polypropylène et un plastifiant, **caractérisé en ce qu**'il contient exclusivement les composants suivants :
1. comme copolymère en masse, 1 partie de SEBS ou 1 partie de SPBS
2. comme adjuvant 0,1 à à partie de copolymère aléatoire de polypropylène contenant comme monomère de l'éthylène ou du butylène à une concentration de 2% à 8%
3. comme plastifiant 0,1 à à partie d'une huile contenant au maximum 10% de constituants aromatiques et
4. éventuellement un antistatique pour une part de 0,1% à 0,5% en poids,
la fabrication du composé d'élastomère se faisant en excluant tout réticulation chimique.

2. Composé selon la revendication 1, **caractérisé en ce que** le plastifiant qu'il contient est une huile avec 25% à 45% de constituants naphténiques et 55% à 75% de constituants paraffiniques et libre de constituants aromatiques.

3. Composé selon la revendication 2, **caractérisé en ce que** le plastifiant qu'il contient est de la paraffine perliquide selon DAB 10.

4. Articles médicaux pour les secteurs suivants : infusion, transfusion, dialyse, alimentation parentérale, laparoscopie et prélèvement sanguin, **caractérisés en ce qu'**ils peuvent être réalisés par extrusion ou moulage par injection d'un composé selon les revendications 2 à 3.

5. Articles médicaux selon la revendication 4, **caractérisés en ce qu**'il s'agit de tuyaux flexibles ou de systèmes de tuyaux flexibles.
